# EUROPÄISCHE PATENTSCHRIFT

(11) **EP 2 297 023 B1**
(45) Veröffentlichungstag und Bekanntmachung des Hinweises auf die Patenterteilung: **24.09.2014**
(21) Anmeldenummer: 09772136.9
(22) Anmeldetag: 30.06.2009
(51) Int. Cl.: B67C 3/30, B67C 7/00, A61L 2/20, A61L 2/22, B65B 55/24, B65B 55/10

(54) **VERFAHREN ZUR BEHANDLUNG VON KUNSTSTOFF-EINWEGBEHÄLTERN**
METHOD FOR TREATING DISPOSABLE PLASTIC CONTAINERS
PROCÉDÉ DE TRAITEMENT DES CONTENEURS EN MATIÈRE PLASTIQUE À USAGE UNIQUE

(30) Priorität: 04.07.2008 DE 102008031369
(43) Veröffentlichungstag der Anmeldung: 23.03.2011
(73) Patentinhaber: KHS GmbH, 44143 Dortmund (DE)
(72) Erfinder: MONZEL, Alois, 67591 Mörstadt (DE)
(86) Internationale Anmeldenummer: PCT/EP2009/004690
(87) Internationale Veröffentlichungsnummer: WO 2010/000436

(56) Entgegenhaltungen:
- WO-A-01/28863
- WO-A-79/01074
- WO-A-2006/092367
- WO-A-2007/134803
- WO-A-2007/140883
- WO-A-2008/014991
- DE-A1- 3 502 242
- DE-A1-102004 029 803
- DE-A1-102006 026 279
- GB-A- 2 138 412
- US-A- 3 392 034
- US-A1- 2004 208 781

## Beschreibung

Die Erfindung betrifft ein Verfahren zur Behandlung von Kunststoff-Einwegbehältern, insbesondere von Kunststoff-Einweggroßbehältern wie Einweg-Kegs, im Zuge ihrer Befüllung, wonach ein Sterilisationsmedium mit zu reinigenden Oberflächen in Kontakt gebracht wird, - Bei Kunststoff-Elnweggroßbehältern handelt es sich üblicherweise um solche Behälter mit einem Inhalt in der Größe von 30 Litern oder auch 50 Litern. Jedenfalls beträgt der Volumeninhalt solcher Kunststoff-Einweggroßbehälter durchweg mehr als 10 Liter.

Der Einsatz von Kunststoff-Einwegbehältern insbesondere in der Getränkeindustrie, also zur Aufnahme von Getränken, nimmt ständig zu. Hierfür gibt es mehrere Gründe. An erster Stelle steht zunächst einmal die im Vergleich zu Behältern aus Glas deutlich einfachere Handhabung aufgrund des verringerten Gewichts. Als Folge hiervon sind auch die Transportkosten niedriger.

Außerdem verlangen die Endverbraucher zunehmend ein geringeres Gewicht der abgefüllten Behälter, um die körperlichen Belastungen auf ein Minimum zu reduzieren. Das gilt auch und insbesondere für Kunststoff-Einweggroßbehälter sowie Einweg-Kegs. Diese kommen zunehmend bei der Belieferung von Gaststätten durch Brauereien zum Einsatz. Neben den Vorteilen der einfacheren Handhabung und des geringeren Gewichtes kann hier noch als weiterer Vorteil geltend gemacht werden, dass sich Einweg-Kegs nach ihrer Entleerung zusammendrücken oder -falten lassen, das heißt, ein im Vergleich zum abgefüllten Zustand deutlich verringertes Volumen aufweisen. Außerdem sind Kosten gering. Bisher werden solche Einweg-Kegs unmittelbar entsorgt, wenngleich grundsätzlich natürlich auch ein Recycling im Sinne eines Pfandsystems möglich ist.

Bei der Abfüllung von flüssigen Lebensmitteln und hier insbesondere Getränken muss besonders deren mikrobiologische Qualität beibehalten werden, Zu diesem Zweck wird regelmäßig im Stand der Technik, wie er beispielsweise in der DE 44 27 577 C2 beschrieben wird, so vorgegangen, dass die fraglichen Behälter thermisch sterilisiert werden, um eventuell vorhandene und für das jeweilige Lebensmittel schädliche Keime abtöten zu können. Um dies zu erreichen wird beispielsweise Dampf oder Heißwasser in eine zu sterilisierende Flasche eingeleitet. - Die insofern bekannte Vorgehensweise lässt sich jedoch nur bei Mehrweg-Kegs aus Aluminium oder beispielsweise Flaschen aus Glas einsetzen. Für Kunststoff-Einwegbehälter eignet sich diese Vorgehensweise nicht oder kaum.

Das lässt sich auf die Temperaturempfindlichkeit der eingesetzten Kunststoffmaterialien bei der Herstellung solcher Kunststoff-Einwegbehälter zurückführen. Denn die mangelnde Temperaturstabilität ermöglicht nur sehr eingeschränkt eine Behandlung mit Wärme oder allgemein ein Pasteurisieren. So dürfen z. B. aus PET bestehende Kunststoff-Einwegbehälter nicht über 60° C erwärmt werden. Denn eine nur kurzzeitige Erwärmung auf Werte von mehr als 70° C führt oftmals zur Zerstörung des Behälters.

Aus diesem Grund werden Kunststoff-Einwegbehälter In der Praxis häufig mittels eines so genannten Rinsverfahrens unter Zusatz von Desinfektionsmitteln in einen so genannten getränkesterilen Zustand gebracht. Dieser getränkesterile Zustand drückt aus, dass der fragliche Kunststoff-Einwegbehälter nach der beschriebenen Vorgehensweise unmittelbar mit dem flüssigen Lebensmittel befüllt werden kann.

Bei den aus der Praxis bekannten Rinsverfahren werden die Kunststoff-Einwegbehäiter mit ihrer Mündung nach unten orientiert. Durch diese Mündung wird die zugehörige Spülflüssigkeit bzw. das Desinfektionsmittel mit einer solchen Menge und Geschwindigkeit in den Kunststoff-Einwegbehälter gespritzt, dass der Behälterboden sicher erreicht wird. Zum Abschluss wird mit einer Spülflüssigkeit gespült, welche die Innenwände benetzt um diese zu reinigen. Die Spülflüssigkeit läuft bei diesem Vorgang durch die Mündung wieder aus dem Kunststoff-Einwegbehälter heraus.

Ebenfalls sind in der Praxis auch solche Rinsverfahren bekannt geworden, welche auch gasförmige Sterilisationsmedien vorsehen, doch sieht der gesamte Stand der Technik ausschlleßlich die Anwendung solcher gasförmiger Sterilisationsmedien für Behälter ohne eine Innenreinigung erschwerende, aufwendige Anschlussarmaturen wie an Kegs verwendete Fittings vor. So spricht beispielsweise die EP 0 722 741 A2, ausschließlich von mit einem Giebeldach versehen Kartonverpackungen, wobei diese noch unverschlossen sind. Auch die DE 10 2004 029 803 B4 spricht ausschließlich und allgemein von der Sterilisation von Flaschen, Dosen und Behältern, wobei sich die Ausführungsbeispiele dieser Schrift ausschließlich auf Flaschen beziehen. Auch die DE 10 2006 026 276 B1 spricht ausschließlich und allgemein von der Sterilisation von Flaschen, Dosen und Behältern, wobei sich die Ausführungsbeispiele dieser Schrift ausschließlich auf Flaschen beziehen.

Die US 5,843,374 befasst sich ausschließlich mit der Sterilisation von Behältern, welche zumindest teilweise aus heißsiegelbarem Polymermaterial bestehen. Die EP 0 411 970 A1, befasst sich mit der Sterilisation der Oberflächen von Behältern, die mit Lebensmitteln in Berührung kommen, wobei nähere Angaben zu den Behältern nicht gemacht werden.

Die DE 35 02 242 A1 betrifft ein Verfahren zur Reinigung des Innenraums von Kegs gemäß dem Oberbegriff des Anspruchs 1. Dabei wird das Keg vorzugsweise von unten mit Wasser, welches Ozon in gelöster und/oder Gas-Form enthält, zumindest einmal gefüllt. In Anschluss an das Befüllen wird das im Keg befindliche ozonhaltige Wasser mit Ozon-Gas oder ozonhaltigem Gas beaufschlagt und nach seiner letzten Füllung durch Ozon-Gas oder ozonhaltiges Gas aus dem Keg ausgetrieben. Für dieses Verfahren sieht diese Schrift die Verwendung einer eigenständigen Vorrichtung vor. Die Verwendung einer kombinierten Reinigungs- und Füllvorrichtung jedenfalls sieht diese Schrift nicht vor.

Die WO 2007/140883 A1 betrifft ein Verfahren und eine Vorrichtung zum Behandeln von Flaschen oder dergleichen Behälter mit einem Behandlungsmedium. Dabei ist mehr im Detail vorgesehen, dass das Behandlungsmedium nach dem Einbringen in den Behälter durch Beaufschlagung mit elektromagnetischer Strahlung aktiviert wird. Auch diese Schrift sieht für dieses Verfahren die Verwendung einer eigenständigen Vorrichtung vor. Die Verwendung einer kombinierten Reinigungs- und Füllvorrichtung jedenfalls sieht diese Schrift nicht vor.

Die beschriebenen und aus der Praxis bekannten Rinsverfahren eignen sich für Kunststoffflaschen oder dergleichen Behälter, sind jedoch für den Einsatz bei Kunststoff-Einweggroßbehältern wie Einweg-Kegs nicht geeignet. Im Gegensatz zu den Einweg-Kegs weisen die Kunststoffflaschen zum Zeitpunkt des Rinsens eine Behälteröffnung auf, welche frei von Einbauteilen und somit offen ist, was das Einbringen und auch das Auslaufen der Spülflüssigkeit erheblich erleichtert. Einweg-Kegs sind im Gegensatz dazu üblicherweise mit Anschlussarmaturen ausgerüstet, die das beschriebene Rinsen behindern bzw. nur sehr schwer möglich machen. Ein Beispiel für eine solche Anschlussarmatur wird in der DE 31 05 706 A1 beschrieben. Folglich erlauben die Anschlussarmaturen und/oder Einbauten in die Einweg-Kegs ein Rinsen mit vertretbarem Aufwand praktisch nicht, so dass solche Verfahren für Elnweg-Kegs bzw. aligemein Kunststoff-Elnweggroßbehälter praktisch nicht geeignet sind.
Ebenfalls sind aus dem Stand der Technik keine Lösungen bekannt, bei denen Reinigung und Füllung eines Einweg-Kegs Behälters mittels einer Armatur erfolgen, wobei der Behälter mit der Armatur verbunden wird, anschließend Reinigung und Füllung erfolgen, und der Behälter erst im Anschluss an die Füllung wieder von der Armatur getrennt wird.

Hier setzt die Erfindung ein.

Der Erfindung liegt das technische Problem zugrunde, ein Verfahren zur Behandlung von Kunststoff-Einwegbehältern, und hier insbesondere von Kunststoff-Einweggroßbehältern der eingangs beschriebenen Ausgestaltung so weiter zu entwickeln, dass eine einwandfreie Reinigung und Desinfektion unter Berücksichtigung der mangelnden Temperaturstabilität bei geringem Kostenaufwand gegeben ist.

Zur Lösung dieser technischen Problemstellung ist bei einem gattungsgemäßen Verfahren vorgesehen, dass das Sterilisationsmedium durchgängig mit einer deutlich unterhalb einer materialkritischen Temperatur des Kunststofif-Einwegbehälters angesiedelten Temperatur die zu reinigenden Oberflächen beaufschlagt. Dabei hat es sich besonders bewährt, wenn als Sterilisationsmedium ein aktivierbares Sterilisationsgas oder allgemein ein aktivierbares Sterilisationsmedium eingesetzt wird. Die Aktivierung des Sterilisationsmediums bzw. des Sterilisationsgeses wird dabei im Aligemeinen katalytisch und/oder durch äußere Energiezufuhr vorgenommen. Die äußere Energiezufuhr kann beispielsweise durch Licht, Wärme, mechanische Energie etc. erfolgen.

Erfindungsgemäß findet also ein spezielles Sterilisationsmedium Verwendung, nämlich üblicherweise ein aktivierbares Sterilisationsgas. Dieses Sterilisationsgas ist dadurch gekennzeichnet, dass es problemlos über eine Anschlussarmatur bzw. einen in das Keg hineinreichenden so genannten Degen die zu reinigenden Oberflächen insbesondere im innern des Einweg-Kegs und auch im Inneren der Anschlussarmatur erreicht. Dadurch, dass das Sterilisationsmedium bzw. Sterilisationsgas von außen aktiviert wird, findet letztlich keine thermische Sterilisation statt, sondern eine kalte Sterilisation, die mit chemischen Zersetzungsprozessen einhergeht.

Tatsächlich zerfällt nämlich das Sterilisationsmedium bzw. Sterilisationsgas im Zuge seiner Aktivierung in wenigstens eine Sterilisationskomponente und eine Restkomponente. Als Sterilisationsgas empfiehlt die Erfindung den Einsatz von beispielsweise Ozon oder auch Wasserstoffperoxid. Beide Gase bzw. Sterilisationsgase können durch eine Aktivierung in üblicherweise freie Radikale, nämlich atomaren Sauerstoff und HO-Gruppen aufgespalten werden. Dabei zeichnen insbesondere die freien Sauerstoffradikale als Sterilisationskomponente für die keimabtötende Wirkung auf den entsprechend beaufschlagten Oberflächen verantwortlich. Die Restkomponente (meistens Wasser) ist dagegen unschädlich, was die anschließende Befüllung mit dem flüssigen Lebensmittel angeht.

Die Aktivierung des Sterilisationsmedlums bzw. des Sterilisationsgases kann vor und/oder während und/oder nach seinem An- oder Auftragen auf die zu behandelnden Oberflächen erfolgen. Das hängt davon ab, mit welcher Aktivierungsmethode gearbeitet wird. So ist es grundsätzlich aus anderem Zusammenhang bekannt (vgl. DE 10 2004 029 803 B4), das Sterilisationsgas bzw. Sterilisationsmedium so zu aktivieren, dass in seinem Strömungsweg wenigstens ein die Zersetzung des Sterilisationsmediums fördernder Katalysator angeordnet ist. In diesem Fall wird das Sterilisationsmedium vor seinem An- oder Auftreffen auf die zu reinigende Oberfläche aktiviert.

Darüber hinaus ist es denkbar, das Sterilisationsmedium durch beispielsweise UV-Licht während und/oder nach seinem An- oder Auftreffen auf die zu reinigende Oberfläche zu aktivieren. Tatsächlich kann das fragliche UV-Licht relativ problemlos ins Innere eines solchen Kunststoff-Einweggroßbehälters eingekoppelt werden, entweder direkt durch die für UV-Licht transparente Kunststoffwandung oder beispielsweise mit Hilfe eines durch die Anschlussarmatur hindurchgeführten Lichtleiters oder mehrerer solcher Lichtleiter. Darüber hinaus ist es denkbar, beispielsweise eine UV-Lampe im Strömungsweg anzuordnen. Alternativ oder zusätzlich kann die Aktivierung auch durch eine Behandlung mit Ultraschall und/oder durch Mikrowellen vorgenommen werden. Beide Energieformen lassen sich üblicherweise von außen problemlos ins Innere eines Kunststoff-Einweggroßbehälters einkoppeln.

Um eine vollständige und großflächige Reinigung bzw. Desinfektion des Kunststoff-Einwegbehälters zu erzielen, empfiehlt es sich, sowohl Innen- als auch gegebenenfalls Außenflächen des fraglichen Kunststoff-Einwegbehälters zu reinigen. Darüber hinaus kann optional auch die Anschlussarmatur eine Reinigung mittels des Sterilisationsmediums erfahren. Das heißt, der Kunststoff-Einwegbehälter erfährt eine vollständige Reinigung von Innen und zumindest der Außenflächen im Mündungsbereich. Darüber hinaus kann dann noch die Anschlussarmatur mit ihren Dichtungen und Dichtflächen desinfiziert bzw. gereinigt werden. Nach der Reinigung wird dann vorteilhaft die fragliche Anschlussarmatur in die Mündung des Kunststoff-Einwegbehälters eingesetzt und mit diesem verbunden oder sie ist bereits in der Mündung vorhanden und hat die Reinigung erfahren.

Nach Abschluss des Sterilisationsvorganges wird der Kunststoff-Einwegbehälter mit einem Reinigungsmedium gespült. Bei dem Reinigungsmedium handelt es sich verteilhaft um ein Vorspanngas, welches anschließend auch für die erforderliche Vorspannung im Innern des Kunststoff-Einwegbehälters sorgt, damit zum Abschluss das einzufüllende flüssige Lebensmittel unter Vorspannung in den Kunststoff-Einwegbehälter eingefüllt werden kann.

So oder so gelingt es im Rahmen der Erfindung erstmals, insbesondere Kunststoff-Einweggroßbehälter aus einem temperaturempfindlichen Kunststoffmaterial getränkesteril abzufüllen. Das erreicht die Erfindung dadurch, dass auf ein spezielles Sterilisationsmedium zurückgegriffen wird, welches den fraglichen Kunststoff-Einwegbehälter mit einer deutlich unterhalb der materialkritischen Temperatur angesiedelten Temperatur beaufschlagt. Die Arbeit in dem niedrigen Temperaturbereich gelingt, weil das Sterilisationsmedium seine Wirkung bereits im Bereich der zulässigen Temperaturen entfaltet. Eine eventuell erforderliche Aktivierung des Sterilisationsmediums erfolgt vorzugsweise nicht durch Hitze, insbesondere nicht durch Erwärmung des gesamten Kunststoff-Einwegbehälters, sondern durch einen (kalten) chemischen Zersetzungsprozess der von außen initilert wird, oder durch einen gezielten Wärmeeintrag in das Sterilisationsmedium.

Insgesamt sorgt das Einleiten des Sterilisationsgases mit dem gewünschten Sterilisationseffekt dafür, dass der Kunststoff-Einwegbehälter auf diese Weise hinsichtlich etwaiger vorhandener Keime gereinigt und steril gemacht wird. Dabei stellt der Rückgriff auf beispielsweise Ozon oder Wasserstoffperoxid als denkbares Sterilisationsgas sicher, dass seine Anwendung nicht zu einem Anstieg der Sauerstoffkonzentration im Keg führt. Hierfür sorgt auch der nach dem Sterilisationsvorgang obligatorische Spülvorgang mit dem Reinigungsmedium.

Dadurch, dass vorteilhaft ein Sterilisationsgas zum Einsatz kommt, lässt sich das Sterilisationsmedium problemlos über die ohnehin vorhandene bzw. obligatorische Anschlussarmatur in den Kunststoff-Einweggroßbehälter einleiten. Das geschieht meistens über einen so genannten Degen. Gleichzeitig sorgt ein Ringkanal bei einer solchen Anschlussarmatur dafür, dass das fragliche Sterilisationsgas aus dem Kunststoff-Einweggroßbehälter einwandfrei austreten kann. Der Aufbau einer solchen Armatur ist beispielhaft - wie bereits genannt - der DE 31 05 706 A1 zu entnehmen.

Insgesamt werden alle Oberflächen, die mit dem abzufüllenden flüssigen Lebensmittel in Berührung kommen, einwandfrei sterilisiert. Zu diesen Oberflächen gehören auch Bestandteile und Oberflächen der Anschlussarmatur bzw. eines Fittings, da dieses während der Reinigungsphase geöffnet ist, so dass das hindurchströmende Sterilisationsmedium bzw. Sterilisationsgas sämtliche relevanten Oberflächen, Dichtungen, Dichtungsflächen etc. erreicht.

Die Dauer der Sterilisationsbehandlung ist dabei von der Sterilisationswirkung des verwendeten Sterilisationsgases abhängig und selbstverständlich auch davon, wie intensiv und schnell die Aktivierung des Sterilisationsgases durchgeführt wird. Jedenfalls findet nach Abschluss des Sterilisationsprozesses eine Spülung mit dem Reinigungsmedium statt, bei dem es sich vorteilhaft um ein Vorspanngas, insbesondere CO₂, handelt. Auf diese Weise kann das Reinigungsmedium unmittelbar als Vorspanngas bei der abschließenden Abfüllung des flüssigen Lebensmittels, beispielsweise Bier, Verwendung finden. Hierin sind die wesentlichen Vorteile zu sehen.

Im Folgenden wird die Erfindung anhand einer lediglich ein Ausführungsbeispiel darstellenden Zeichnung näher erläutert; es zeigen:
- Fig. 1: eine geeignete Vorrichtung im Ausschnitt und
- Fig. 2: eine Übersicht über die geeignete Vorrichtung.

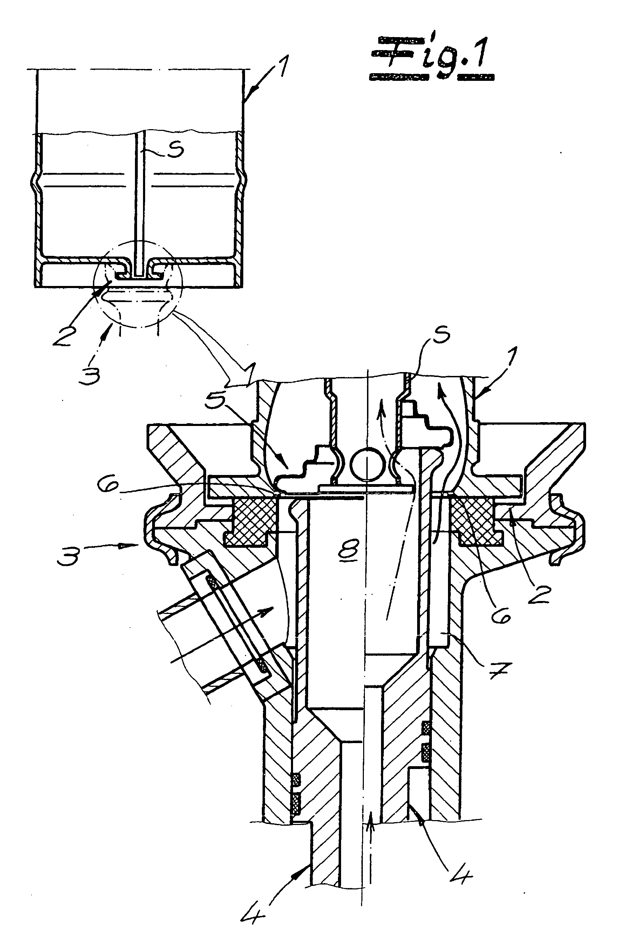

In der Fig. 1 ist ausschnittsweise ein Kunststoff-Einweggroßbehälter in Gestalt eines Einweg-Kegs 1 dargestellt. Das Einweg-Keg 1 Ist aus Kunststoff, beispielsweise PET (Polyethylenterephthalat) gefertigt. Das gilt selbstverständlich nur beispielhaft und nicht einschränkend. Jedenfalls dient das Einweg-Keg 1 allgemein zur Aufnahme von flüssigen Lebensmitteln, vorliegend zur Aufnahme von Getränken, speziell von Bier. Dazu wird das Einweg-Keg 1 in eine in Fig. 1 dargestellte Überkopfposition verbracht und wird eine dortige Ventilarmatur 2 mit einem Füllkopf 3 verbunden. In dem Füllkopf 3 findet sich ein axial verschiebbarer Stößel 4, der entsprechend der Darstellung in Fig. 1 geschlossen und geöffnet werden kann.

Mit Hilfe des Stößels 4 lässt sich ein Ventil 5 innerhalb des Füllkopfes 3 von einem zugehörigen Ventilsitz 6 abheben, wie es Im rechten Teil der Fig. 1 dargestellt ist. Auf diese Weise kann ein Produktmedium, im vorliegenden Fall die Flüssigkeit respektive das Getränk oder Bier, über einen äußeren Ringkanal bzw. Produktkanal 7 in das Fassinnere strömen. Das ist durch entsprechende Pfeile angedeutet.

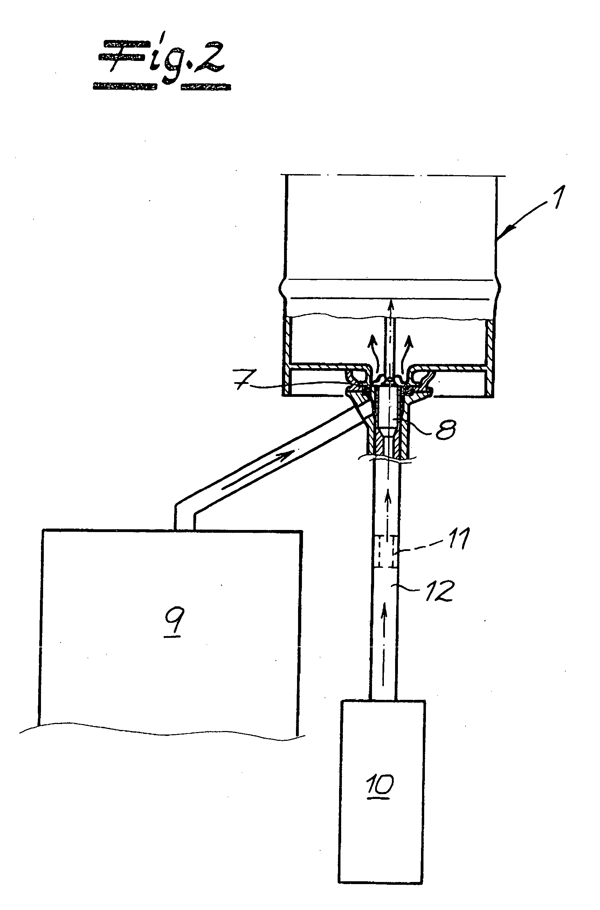

Wie die Übersichtdarstellung gemäß Flg. 2 deutlich macht, wird das Bier bzw. allgemein das Getränk in einem Vorratsbehälter 9 für das Getränk bevorratet, welcher mit dem Ringkanal 7 kommuniziert. Darüber hinaus erkennt man noch einen Gaskanal 8 für ein Vorspanngas, der durch das Innere des hohlzylindrisch ausgeführten Stößels 4 verläuft. Der Gaskanal 8 setzt sich in einem Steigrohr respektive Steckdegen S fort, welches bzw, welcher ins Innere des Einweg-Kegs 1 ragt. In der rechten Darstellung gemäß Fig. 1 sind sowohl der äußere Ringkanal bzw. Produktkanal 7 als auch der Gaskanal 8 geöffnet.

Über den Gaskanal 8 kann nun vor dem Befüllen mit dem flüssigen Lebensmittel bzw. Bier ein Sterilisationsmedium in das dargestellte Einweg-Keg 1 zugeführt werden. Dazu ist ein weiterer Vorratsbehälter 10 für das Sterilisationsmedium realisiert, über den dieses das Innere des Einweg-Kegs 1 erreicht.

Erfindungsgemäß beaufschlägt das Sterilisationsmedium die zu reinigenden Oberflächen im Innern des Einweg-Kegs 1 und auch im Bereich einer dort realisierten Anschlussarmatur 2, 3 aus der Ventilarmatur 2 und dem Füllkopf 3. Dies geschieht praktisch auf kaltem Weg. nämlich durch eine chemische Zersetzungsreaktion. Tatsächlich kommt als Sterilisationsmedium ein aktivierbares und im Vorratsbehälter 10 befindliches Sterilisationsgas zum Einsatz, welches während seiner gesamten Behandlung des Einweg-Kegs 1 wie der Anschlussarmatur 2, 3 eine Temperatur aufweist, die unterhalb einer materialkritischen Temperatur des Einweg-Kegs 1 angesiedelt ist. Die materialkritische Temperatur liegt im vorliegenden Fall und bei Verwendung eines Kunststoffes wie beispielsweise PET für die Herstellung des Einweg-Kegs 1 jedenfalls unterhalb von 50° C, meistens sogar noch unterhalb von 40° C. Das ist erforderlich, um Schädigungen des Kunststoffs auf jeden Fall ausschließen zu können.

Zur Aktivierung des eingesetzten Sterilisationsmediums bzw. Sterilisationsgeses ist eine Aktivierungseinrichtung 11 vorgesehen. Hierbei mag es sich um einen Katalysator 11 handeln, der in einen Zuführungskanal 12 für das aus dem Vorratsbehälter 10 abgezogene Sterilisationsgas eingebracht worden ist. Selbstverständlich kann die Aktivierungseinrichtung 11 auch als UV-Lampe, Mikrowellengenerator etc. ausgestaltet sein. Das hängt davon ab, auf welche Weise die Aktivierung des Sterilisationsmediums bzw. Sterilisationsgases stattfinden soll.

Nach der Sterilisation des Einweg-Kegs 1 sowie der Anschlussarmatur 2, 3 werden die mit dem Sterilisationsgas jeweils gereinigten Oberflächen mit Hilfe eines Reinigungsmediums gespült. Bei dem Reinigungsmedium handelt es sich um ein Vorspanngas, im Ausführungsbeispiel CO₂. Dieses Vorspanngas wird über den bereits angesprochenen Gaskanal 8 zugeführt. Sobald das Innere des Einweg-Kegs vorgespannt ist, kann das flüssige Getränk aus dem Vorratsbehälter 9 über den Ringkanal bzw. Produktkanal 7 zugeführt werden.

Der dargestellten Vorrichtung zur Reinigung bzw. Sterilisation der betreffenden Einweg-Kegs 1 kann grundsätzlich eine Vorrichtung zur Herstellung solcher Einweg-Kegs 1, beispielsweise eine Streckblasmaschine vorgeordnet werden. Grundsätzlich lässt sich die Streckblasmaschine auch mit der beschriebenen Vorrichtung kombinieren.

Die vorliegende Erfindung erstreckt sich auch auf Verfahren, bei denen als Sterilisationsmedium ein Aerosol verwendet wird. Dabei ist es selbstverständlich, dass alle Bestandteile des Aerosols, zumindest aber dessen Abbauprodukte, ernährungsphysiologisch unbedenklich sind, und somit zur Sterilisation von Getränkebehältern verwendet werden können.

## Patentansprüche

1. Verfahren zur Behandlung von Kunststoff-Einweg-Kegs (1), im Zuge ihrer Befüllung, wobei die Kunststoff-Einweg-Kegs bereits mit ihrer Anschlussarmatur (2, 3) versehen sind, wonach über die bereits mit dem **Kunststoff-Einweg-Keg** verbundene Anschlussarmatur (2,3) ein Sterilisationsmedium mit zu reinigenden **Inneren** Oberflächen in Kontakt gebracht wird, **wobei** es sich bei dem Sterilisationsmedium um ein katalytisch und/oder durch äußeren Energieeintrag aktivierbares Gas oder Aerosol handelt, wobei das Sterilisationsmedium die Oberflächen des Kunststoff-Einwegbehälters mit Temperaturen beaufschlägt, welche unterhalb einer materialkritischen Temperatur des Kunststoff-Einwegbehälters liegen, **dadurch gekennzeichnet, dass** der Kunststoff-Einwegbehälter nach der Beendigung der Sterilisationsvorganges über die bereits mit dem Kunststoff-Einweg-Keg verbundene Anschlussarmatur (2,3) mit einem Reinigungsmedium gespült und vorgespannt wird und abschließend Ober die bereits mit dem Kunststoff-Einweg-Keg verbundene Anschlussarmatur (2,3) eine Flüssigkeit unter Vorspannung eingefüllt wird.

2. Verfahren nach Anspruch 1, **dadurch gekennzeichnet, dass** als Sterilisationsmedium ein aktivierbares Sterilisationsgas eingesetzt wird.

3. Verfahren nach einem der Ansprüche 1 oder 2, **dadurch gekennzeichnet, dass** das Sterilisationsmedium vor und/oder während und/oder nach seinem An- oder Auftreffen auf die zu reinigenden Oberflächen aktiviert wird.

4. Verfahren nach einem der Ansprüche 1 bis 3, **dadurch gekennzeichnet, dass** das Sterilisationsmedium im Zuge seiner Aktivierung in wenigstens eine Sterilisationskomponente und eine Restkomponente chemisch zerfällt.

5. Verfahren nach einem der Ansprüche 1 bis 4, **dadurch gekennzeichnet, dass** sowohl Innen- als auch Außenflächen des Kunststoff-Einwegbehälters sowie Anschlussarmaturen (2, 3) mittels des Sterilisationsmediums gereinigt werden.

## Claims

1. Method for treating plastic disposable kegs (1) in the course of the filling thereof, wherein the plastic disposable kegs are already fitted with a connection armature (2, 3), whereby a sterilisation medium is brought into contact with internal surfaces to be cleaned by means of the connection armature (2, 3) already connected to the plastic disposable keg, wherein the sterilisation medium is a catalytic gas or aerosol and / or a gas or aerosol activated by an external energy furnish, wherein the sterilisation medium acts on the surfaces of the plastic disposable container at temperatures which are below a material-critical temperature of the plastic disposable container, **characterised in that** the plastic disposable container is rinsed and pre-stressed with a cleaning medium after the end of the sterilisation process by means of the connection armature (2, 3) already connected to the plastic disposable keg, and then filled with a liquid under pre-stress by means of the connection armature (2, 3) already connected to the plastic disposable keg.

2. Method according to claim 1, **characterised in that** an activatable sterilisation gas is used as the sterilisation medium.

3. Method according to any one of claims 1 to 2, **characterised in that** the sterilisation medium is activated before and / or during and / or after it comes into contact with or impinges on the surfaces to be cleaned.

4. Method according to any one of claims 1 to 3, **characterised in that** the sterilisation medium chemically decomposes into at least one sterilisation component and one residual component in the course of its activation.

5. Method according to any one of claims 1 to 4, **characterised in that** both internal and external surfaces of the plastic disposable container and connection armatures (2, 3) are cleaned with the sterilisation medium.

## Revendications

1. Procédé de traitement de fûts en matière plastique à usage unique (1) au cours de leur remplissage, les fûts en matière plastique à usage unique étant déjà pourvus de leur armature de raccordement (2, 3), après quoi un agent de stérilisation est amené en contact avec des surfaces intérieures à nettoyer par le biais de l'armature de raccordement (2, 3) déjà reliée au fût en matière plastique à usage unique, l'agent de stérilisation étant un gaz ou un aérosol activable par voie catalytique et/ou par apport d'énergie extérieur, l'agent de stérilisation sollicitant les surfaces du fût en matière plastique à usage unique avec des températures qui se trouvent sous une température critique pour le matériau du fût en matière plastique à usage unique, **caractérisé en ce que** le fût en matière plastique à usage unique est rincé et précontraint par un agent de nettoyage à la fin du processus de stérilisation par le biais de l'armature de raccordement (2, 3) déjà reliée au fût en matière plastique à usage unique et un liquide est ensuite introduit sous précontrainte par le biais de l'armature de raccordement (2, 3) déjà reliée au fût en matière plastique à usage unique.

2. Procédé selon la revendication 1, **caractérisé en ce qu'**un gaz de stérilisation activable est utilisé comme agent de stérilisation.

3. Procédé selon l'une quelconque des revendications 1 ou 2, **caractérisé en ce que** l'agent de stérilisation est activé avant et/ou pendant et/ou après sa rencontre avec les surfaces à nettoyer ou son arrivée sur celles-ci.

4. Procédé selon l'une quelconque des revendications 1 à 3, **caractérisé en ce que** l'agent de stérilisation se désintègre chimiquement au cours de son activation en au moins un composant de stérilisation et un composant résiduel.

5. Procédé selon l'une quelconque des revendications 1 à 4, **caractérisé en ce que** des surfaces intérieures ainsi que des surfaces extérieures du fût en matière plastique à usage unique et des armatures de raccordement (2, 3) sont nettoyées à l'aide de l'agent de stérilisation.
